# EUROPEAN PATENT SPECIFICATION

(11) **EP 2 537 422 B1**
(45) Date of publication and mention of the grant of the patent: **21.10.2015**
(21) Application number: 12173112.9
(22) Date of filing: 22.06.2012
(51) Int. Cl.: A23L 1/22, A23L 1/236, A23L 1/30, A61K 31/7004, A61P 3/04, A61P 43/00, A23L 2/60, A23L 1/09

(54) **Lifespan extending agent**
Wirkstoff zur Verlängerung der Lebensdauer
Agent d'extension de la durée de vie

(30) Priority: 24.06.2011 JP 2011140146; 02.03.2012 JP 2012046942
(43) Date of publication of application: 26.12.2012
(73) Proprietor: National University Corporation Kagawa University, Takamatsu-shi, Kagawa 760-8521 (JP); Matsutani Chemical Industry Co., Ltd., Itami-shi, Hyogo 664-8508 (JP); Izumoring Co. Ltd., Kagawa 761-0615 (JP)
(72) Inventor: Sato, Masashi, Kita-gun, Kagawa 761-0795 (JP); Okuma, Kazuhiro, Itami-shi, Hyogo 664-8508 (JP); Izumori, Ken, Kita-gun, Kagawa 761-0615 (JP)
(74) Representative: TBK

(56) References cited:
- EP-A1- 2 111 865
- WO-A1-2008/056452

## Description

### BACKGROUND OF THE INVENTION

### [Field of the Invention]

The present invention relates to lifespan extending agents of a sugar composition usable as a sweetener. More specifically, the invention relates to a lifespan extending agent of a sugar composition containing D-psicose and/or D-allose usable as sweeteners.

### [Description of the Related Art]

Aging has become a serious social concern in Japan and other developed countries. Among the various factors associated with the control of lifespan and aging, caloric restriction is the most promising for lifespan extension, and the effect of caloric restriction has been confirmed in a wide range of biological species from nematodes to mammals (see, for example, Youichi Nabeshima, The Biology of Aging, Medical Science International (2000) p. 287). However, caloric restriction means reducing the daily food intake, and is not a realistic approach.

Despite numerous studies, lifespan extension has not been fully understood. It has been suggested that the aging phenomenon delaying effect is not synonymous with the lifespan extending effect. For example, it has been shown through animal experiments that vitamin E has the effect of delaying the aging phenomena. However, studies directed to finding the expected lifespan extending effect of vitamin E in mice and rats have not found a clear lifespan extending effect in vitamin E (JP-A-8-176006).

There are reports concerning lifespan extension. For example, JP-A-2007-197374 reports that fruit (apple)-derived polyphenol prevents heart failure or congestive heart failure, and extends the lifespan of a model mouse with congestive heart failure. JP-A-2005-210978 reports that oil and fat compositions of specific properties containing α-linolenic acid and linoleic acid can extend the lifespan of rats susceptible to stroke. These lifespan extending effects are found in model mice with specific diseases, and in mice under high-calorie diet, and are believed to extend lifespan by preventing or treating specific diseases or disease conditions.

Compositions containing a specific chitosan (JP-A-2005-289839), peroxidase (JP-A-5-124980), or activated carbon (JP-A-2010-208969) as active ingredients are reported as compositions capable of extending the lifespan of normal individuals. The activated carbon is not a compound absorbable by an organism. The lifespan extending effects of the chitosan and peroxidase are not prominent (JP-A-2010-208969).

EP 2 111 865 A1 is directed at the utilization of the function of rare sugars, e.g. composition comprising psicose and tagatose, in the treatment of disorders associated with glucokinase activity.

WO 2008/056452 A1 describes an agent containing D-allose as an active ingredient for slowing the onset or progress of mobility impairment relating to amylotropic lateral sclerosis.

### SUMMARY OF THE INVENTION

Accordingly, there is a need for a lifespan extending agent that can safely and effectively extend lifespan even when used continuously in a form that resembles food. In the present invention, lifespan extension is realized by giving rare sugars, such as D-psicose and D-allose, used as sweeteners to animals.

It is well known that dieting by intake calorie restriction extends the lifespan of animals such as nematodes, flies, mice, and monkeys. It is also known that dieting by intake calorie restriction delays the onset of age-related diseases, including, for example, diabetes mellitus, cancer, and Alzheimer's disease, and extends the lifespan of humans. In reality, however, dieting by intake calorie restriction is difficult to sustain for extended time periods, and there is a need for some means to imitate the intake calorie restriction dieting and obtain the same effects. The present invention has been made to provide a useful technique that solves the foregoing problem based on the same mechanism of lifespan extension found in the related art.

The present inventors completed the present invention based on the finding that a composition that contains a rare sugar usable as a sweetener contributes to lifespan extension as an active ingredient.

Specifically, the gist of the present invention includes **the following lifespan extending agents (1) to (6) for the use as defined in claim 1.**
**(1) An agent comprising a rare sugar usable as a sweetener as an active ingredient for use in extending lifespan by enhancing the production or activity of superoxide dismutase (SOD) and catalase.**
**(2) The agent according to (1),** wherein the rare sugar is D-psicose and/or D-allose.
(3) The agent according to (1), wherein the rare sugar is a sugar composition containing D-psicose and/or D-allose.
(4) The agent according to (3), wherein the rare sugar is a sugar composition containing 0.5 to 17.0% D-psicose and 0.2 to 10.0% D-allose with respect to the sugar content.
(5) The agent according to (4), wherein the rare sugar is a mixed sugar produced by the conversion of raw material sugars D-glucose and/or D-fructose to include 0.5 to 17.0% D-psicose and 0.2 to 10.0% D-allose with respect to the total sugar content.
(6) The agent according to (4) or (5), wherein the rare sugar is a rare sugar-containing isomerized sugar usable as a sweetener.
**(7) The lifespan extending agent according to any one** of (1) to **(6),** wherein lifespan is extended based on the same mechanism of lifespan extension that operates in an intake calorie restricted state.

The present invention can provide a lifespan extending agent that contains a rare sugar usable as a sweetener, preferably D-psicose and/or D-allose, as an active ingredient, and that can safely and effectively extend lifespan even when used continuously in a form that resembles food.

More specifically, a lifespan extending agent can be provided that has a sugar composition usable as a sweetener. The sugar composition is a mixed sugar produced by the conversion of raw material sugars of D-glucose and/or D-fructose to a composition including 0.5 to 17.0% D-psicose and 0.2 to 10.0% D-allose with respect to the total sugar content. The D-psicose and/or D-allose are contained as active ingredients.

The lifespan extending agent **extends** lifespan by enhancing the production or activity of superoxide dismutase (SOD) and catalase.

Further, the lifespan extending agent can extend lifespan based on the same mechanism that operates in intake calorie restriction dieting.

### BRIEF DESCRIPTION OF THE DRAWINGS

FIG. 1 is a graph representing the survival rate of the nematode N2 treated with a composition containing 0.25% D-psicose.
FIG. 2 is a graph representing the survival rate of the nematode N2 treated with a composition containing 0.5% D-allose.
FIG. 3 is a graph representing the survival rate of the nematode N2 treated with a composition containing 0.5% (w/v) D-psicose.
FIG. 4 is a graph representing the survival rate of the nematode mev-1 treated with a composition containing 0.25% (w/v) D-psicose.
FIG. 5 is a graph representing the effect of D-psicose on SOD gene expression in nematodes N2 and mev-1.
FIG. 6 is a graph representing the effect of D-psicose on SOD activity in nematodes N2 and mev-1.
FIG. 7 is a graph representing the effect of D-psicose on catalase gene expression in nematodes N2 and mev-1.
FIG. 8 is a graph representing the effect of D-psicose on catalase activity in nematode N2.
FIG. 9 is a diagram representing a D-psicose-induced lifespan extending mechanism.

### DESCRIPTION OF THE PREFERRED EMBODIMENT

The present invention is concerned with a lifespan extending agent in which a rare sugar usable as a sweetener is contained as an active ingredient. The rare sugar may be, for example, D-psicose and/or D-allose.

The lifespan extending agent of an embodiment of the present invention extends the lifespan of animals by enhancing the production of, for example, superoxide dismutase (SOD) and catalase. SODs are redox enzymes that dismutate a superoxide anion (·O2-) to oxygen and hydrogen peroxide. SODs have divalent or trivalent metal ions such as copper (II) ions and zinc (II) ions (Cu, ZnSOD), and manganese (III) ions (MnSOD) and iron (III) ions (FeSOD) at the active center, and are localized in large amounts in the cytoplasm (Cu, ZnSOD) and mitochondria (MnSOD). Roles of the SOD include reducing the oxidative stress. SODs exist in three forms, SOD1, SOD2, and SOD3, in humans (and in all mammals and in the majority of vertebrates). SOD1 resides in the cytoplasm, SOD2 in the mitochondria, and SOD3 in the extracellular space. The metal ions at the active center are copper and zinc in SOD1 and SOD3, whereas SOD2 has manganese at the active center. Five forms of SODs exist in nematodes. SOD-1, -5 are present in the cytoplasm, SOD-2, -3 in the mitochondria, and SOD-4 in the extracellular space.

Catalases are redox enzymes that dismutate hydrogen peroxide to oxygen and water, and have iron(III) ions at the active center. Catalases are distributed in a wide range of aerobic organisms, including microorganisms, animals and plants. In animals, catalase exists in large amounts in a cellular organelle called peroxisome in the liver and kidney cells, and in red blood cells. In nematodes, catalase exists in two forms, CTL-1 and CTL-2, which reside in the cytoplasm and peroxisome, respectively.

Considering that the SODs and catalases are commonly found in animals, it is believed that the present invention is advantageous as a lifespan extending agent in a wide range of animals.

Testing conducted in the present invention for lifespan measurement using wild-type C. elegans N2 and oxygen sensitive mutant mev-1 revealed that 0.5% (w/v) D-psicose extended the lifespan by 17% in N2. In mev-1, the expression level of Mn-SOD (sod-3) gene localized in mitochondria had a 1.7-fold increase as measured by real time quantitative RT-PCR. The SOD enzyme activity had a 1. 5-fold and a 1. 3-fold increase in N2 and mev-1, respectively. Further, N2 with 0.5% (w/v) D-psicose had a 1.5-fold increase in the gene expression levels of cytoplasm-localized catalase and peroxisome catalases (ctl-1, ctl-2).

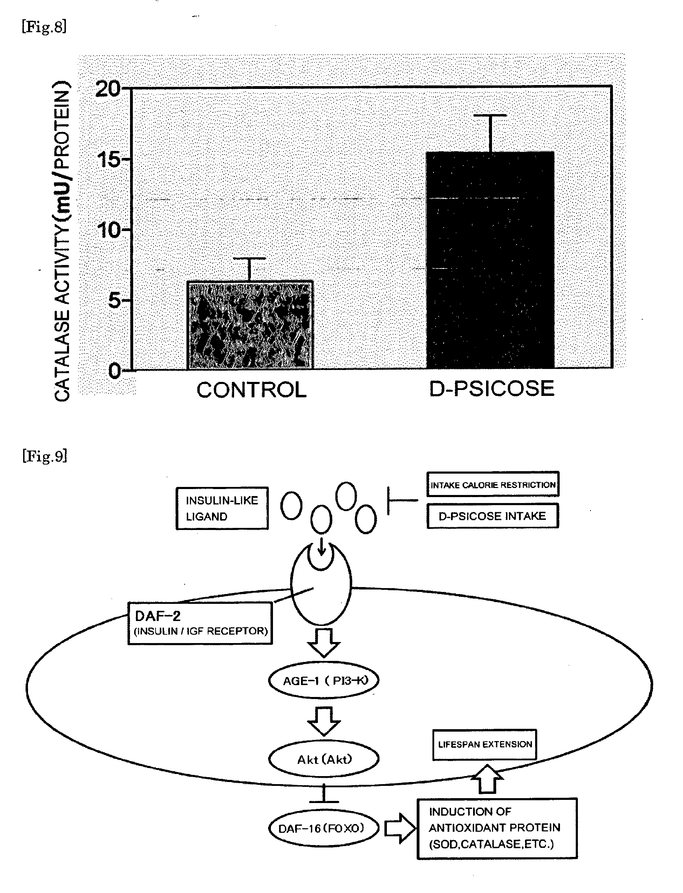

As demonstrated above, D-psicose exhibits the lifespan extending effect by enhancing resistance to oxidative stress. There is a report that the lifespan extension in nematodes in the intake calorie restricted state is induced by antioxidant proteins (such as SODs and catalases) regulated by the insulin/IGF signaling pathway (Braeckman, B.P. and Vanfletren J.R., Exp Gerontol 42, 90-98 (2007)). The SOD and catalase behaviors in response to the rare sugar share the same process as dieting by intake calorie restriction, and the results are the same. It is believed that the lifespan extension by the D-psicose ingestion has the mechanism represented in FIG. 9.

The many compounds act in a similar fashion to intake calorie restricting agents. For example, 2-deoxy-D-glucose inhibits the glycolysis system. Resveratrol, a wine polyphenol, activates sirtuin. The immunosuppresant rapamycin suppresses TOR. The antidiabetic agent metformin activates AMPK. These compounds have not been put into practical applications for reasons including high toxicity, and insufficient effects.

On the other hand, the rare sugars used in the embodiment of the present invention are already in use as edible sweeteners, and thus do not have adverse effects on human body. The rare sugars, with their desirable properties to exhibit high effects, can therefore be easily used in practical applications.

The lifespan extending agent using the rare sugars according to an embodiment of the present invention is described in detail.

### [Rare Sugars]

Among various monosaccharide as the building blocks of sugar, monosaccharides found only in trace amounts in nature are defined as "rare sugars", whereas "natural monosaccharides", as represented by D-glucose (glucose), refer to monosaccharides that are found in large quantities in nature. A total of 59 monosaccharides are known in nature , of which seven are classified as "natural monosaccharides", and fifty two are as "rare sugars". The rare sugars exist only in limited quantities. For example, D-allose is present in much smaller quantities than D-glucose (glucose) in nature.

At present, D-psicose and D-allose are the only rare sugars mass produced. D-psicose is a hexose (C₆H₁₂O₆) present as the D form of psicose classified as a ketohexose in the categories of rare sugars. The D-psicose may be one obtained by different processes, including extraction from nature, chemical syntheses, and biological syntheses. D-allose (D-allohexose) is a hexose (C₆H₁₂O₆) with a melting point of 178°C, present as the D form of allose classified as an aldose (aldohexose). Recently, a method of D-allose production from D-psicose, that is a solution containing the D-psicose is acted upon by D-xylose-isomerase to produce D-allose, is described in JP-A-2002-17392. As described above, D-psicose and D-allose are monosaccharides present in nature only in small amounts. And there is no report pointing out the toxicity of these rare sugars in humans, which suggests the toxicity against animals is low.

D-psicose has a fine, refreshing sweet taste, similar to the sweetness of fructose, absent the discomfort associated with the bitterness and roughness of saccharin. The degree of sweetness is about 70% of sucrose. Further, the non-caloric nature of D-psicose and D-allose has made these rare sugars preferable as healthy sweeteners.

### [Sugar Composition 1 Usable as Sweetener Containing Lifespan Extending Agent Rare Sugar as Active Ingredient]

In one specific form, a sugar composition usable as a sweetener containing a lifespan extending agent rare sugar as an active ingredient is a sweetener that contains about 20 to 80 weight parts of fructose and about 80 to 20 weight parts of glucose and psicose combined, and also contains psicose in a proportion of 5 weight parts or more, preferably 10 weight parts or more with respect to the total 100 weight parts of glucose and psicose in a mixture. The sweetener prevents lifestyle-related diseases such as obesity, and has a degree of sweetness and a taste similar to those of a table sugar (WO2008/142860).

The sweetener is a mixture of fructose, glucose, and psicose components, and is obtained by mixing these components. Fructose and glucose are naturally occurring common monosaccharides, and can be isolated from nature. Fructose also can be separated from, for example, a fructose/glucose liquid sugar obtained by the glucose isomerase treatment of glucose. Glucose is produced from starch by hydrolysis. Psicose, a type of rare sugar present only in limited quantities in nature, can be obtained by treating fructose with ketohexose 3-epimerase. Aside from being produced as a mixture of each constituent component, the sweetener also can be produced by adding D-psicose to a fructose/glucose liquid sugar produced from the raw material glucose acted upon by glucose isomerase, or by converting a part of the fructose into D-psicose in the fructose/glucose liquid sugar acted upon by D-ketose 3-epimerase. Further, the novel sweetener containing glucose, fructose, and D-psicose in the foregoing specific range may be produced in one step from a glucose solution simultaneously acted upon by glucose isomerase and D-ketose 3-epimerase. In terms of cost, it is more advantageous to use a method in which the glucose isomerase treatment (isomerization) and the D-ketose 3-epimerase treatment of the glucose are performed directly in series, or a method in which the target sugar composition is obtained at once from glucose in a mixed enzyme system of glucose isomerase and D-ketose 3-epimerase.

D-psicose is mixed in such a proportion that the ratio of fructose and the total of glucose and psicose ranges preferably from 80 to 20 weight parts:20 to 80 weight parts. With a fructose content of 80 parts or more, sweetness increases but "bodies" decreases. With a fructose content of 20 parts or less, sweetness tends to be not satisfactory. The sweetener exhibits a degree of sweetness and a taste comparable to those of a table sugar when psicose is contained in a proportion of 5 weight parts or more with respect to the total 100 weight parts of glucose and psicose. The sweetener exhibits the obesity preventing effect with a psicose content of 10 weight parts or more.

Various producing methods are possible with the fructose/glucose liquid sugar, glucose, or starch used as the starting raw material.

For example, (1) a fructose/glucose liquid sugar is prepared form starch or glucose, and psicose is produced by the action of ketose 3-epimerase. (2) A decomposed glucose liquid sugar obtained by decomposing starch is acted upon by glucose isomerase and ketose 3-epimerase in a mixed enzyme state.

### (I) Method Using Continuous Plant (Decomposed Fructose/Glucose Liquid Sugar → Ketose 3-Epimerase)

### (1) Production of Decomposed Fructose/Glucose Liquid Sugar Solution

A decomposed fructose/glucose liquid sugar is produced by using an ordinary method, using, for example, a corn, potato, or ocarina starch as the raw material with enzymes such as alphaamylase, glucoamylase, and glucose isomerase in an immobilized or batch system. The raw material starch and the type of the enzyme used are not limited to these. As required, glucose solution production by acidlysis, or isomerization using an alkali may be performed.

### (2) Production of D-Psicose from Fructose/Glucose Liquid Sugar Solution

The decomposed isomerized sugar solution produced is continuously acted upon by epimerase to produce a mixed sugar solution of glucose, fructose, and psicose.

### (II) Method Using Mixed Enzyme (Decomposed Glucose Liquid Sugar → Glucose Isomerase + Ketose 3-Epimerase

Immobilized enzymes including isomerase and epimerase are charged into an appropriate column. Then, a decomposed glucose liquid sugar is continuously flown, and the reaction liquid is removed. Here, the starting substance glucose may be changed to starch, and a mixed enzyme system additionally including alphaamylase and glucoamylase may be used.

For the production of the sugar composition of the embodiment of the present invention, a purified enzyme may be used for the glucose isomerase that acts upon glucose for partial conversion into fructose, or a microorganism producing the enzyme may be used. The ketohexose 3-epimerase is an enzyme that isomerizes the OH at C-3 of a ketohexose such as fructose. Known examples of ketohexose 3-epimerase include D-tagatose 3-epimerase, and D-psicose 3-epimerase (Japanese Patent Number 3333969: D-ketohexose-3-epimerase obtainable from Pseudomonas bacteria; and The 3rd Symposium of International Society of Rare Sugars). The ketohexose 3-epimerase may be a purified enzyme, an immobilized enzyme immobilizing a microorganism producing the enzyme, or an immobilized microorganism.

A common isomerized sugar such as HFCS containing the constituent sugars fructose (42 weight parts) and glucose (58 weight parts) produces glucose (58 weight parts), fructose (34 weight parts), and psicose (8 weight parts) upon being acted upon by tagatose 3-epimerase used as ketohexose 3-epimerase. However, rather unexpectedly, glucose, when acted upon by a mixed enzyme containing glucose isomerase and tagatose 3-epimerase, yielded a mixture of glucose (41 weight parts), fructose (48 weight parts), and psicose (11 weight parts). The both mixtures had a degree of sweetness and a taste that more resembled the table sugar than the fructose/glucose liquid sugar of the related art. The degree of sweetness and the sweet taste can be adjusted by adding fructose and glucose to these mixtures.

### [Sugar Composition 2 Usable as Sweetener Containing Lifespan Extending Agent Rare Sugar as Active Ingredient]

A rare sugar-containing isomerized sugar is an example of the sugar composition that contains D-psicose and D-allose.

Among the sugar mixtures currently in actual use, liquid sugars, or "isomerized sugars" or "High fructose Corn Syrup(HFCS)" as they are often called, containing D-glucose and D-fructose have the highest use for the production of food and beverages. Isomerized sugars include, for example, glucose/fructose liquid sugar, fructose/glucose liquid sugar, and high fructose liquid sugar. These sugars are collectively called isomerized sugars, because, at present, industrial applications are only possible through the isomerization reaction that converts D-glucose (glucose) to D-fructose (fructose) with glucose isomerase. Any mixed sugar produced for the purpose of obtaining a sugar composition containing a specific hexose by taking advantage of the physiological activity of the specific hexose represents a novel sugar composition.

For example, consider a D-psicose- and D-allose-containing isomerized sugar produced by conversion from an "isomerized sugar" broadly regarded as a mixed sugar whose main composition includes D-glucose and D-fructose and targeting D-psicose and D-allose as the hexoses having excellent physiological effects. In this case, the resulting mixed sugar containing the target hexoses in predetermined amounts, and having a sugar composition different from the composition of the raw material sugar represents a novel sugar composition.

A mixed sugar of a desired composition can be produced by treating materials under optimum conditions to satisfy the target hexose content set in advance with respect to the sugar content according to such factors as the type and the extent of the intended functions, usage, and dose. Specifically, an isomerized sugar as a mixed sugar whose main composition includes the raw material sugars D-glucose and D-fructose, or a raw material sugar solution of D-glucose and/or D-fructose is treated in a system that includes at least one selected from the group consisting of a basic ion-exchange resin, an alkali, and a calcium salt. The resulting isomerization reaction (equilibrium reaction) converts the raw material sugars D-glucose and D-fructose into the target D-psicose and D-allose to produce a D-psicose- and D-allose-containing sugar composition of a sugar composition different from the composition of the raw material isomerized sugar, and containing 0.5 to 17.0% D-psicose and 0.2 to 10.0% D-allose with respect to the sugar content (see WO2010/113785). For example, the hexose composition contains both D-psicose and D-allose, preferably with 1.0 to 15.0% D-psicose and 0.4 to 8.0% D-allose, most preferably with about 2.5 to 8.0% D-psicose and 1.5 to 5.0% D-allose with respect to the total sugar content. The hexose composition containing both D-psicose and D-allose has prominent effects, unexpectedly exhibited by the synergy of the two not possible with the D-psicose or D-allose alone. For example, the composition exhibits excellent physiological effects. Specifically, such synergic effects are seen in body weight reduction rate, body fat reduction rate, and diet reduction rate. By the ingestion of the sugar composition containing D-psicose and D-allose, decreases in glucose level and insulin level can be observed. A decrease in insulin level by the sugar composition is the effect not found in the previous testing using D-psicose or D-allose alone.

For the sugar composition to exhibit the excellent effects above, the proportions of D-psicose and D-allose with respect to the total sugar content are preferably 0.5 to 15.0%, more preferably 1.0 to 15.0%, most preferably 2.5 to 8% for D-psicose, and preferably 0.2 to 10.0%, more preferably 0.4 to 8.0%, most preferably 1.5 to 5.0% for D-allose.

The total amount of D-psicose and D-allose ranges from preferably 0.7 to 25.0%, more preferably 1.4 to 23.0%, further most preferably 4.0 to 13.0%.

The sugar composition may be used with sweeteners such as sucrose, sugar alcohol, aspartame, and stevia, as desired. Further, for integrity or other desired properties, the sugar composition may be used by being mixed in water-soluble dietary fibers of low sweetness (such as polydextrose, inulin, and resistant dextrin), or in other physiologically active components according to the intended use or preferences.

### [Use and Application of Lifespan extending agent of Embodiment of the Present Invention]

The lifespan extending agent of the embodiment of the present invention can be used for drugs and quasi drugs, oral compositions, cosmetics, food, food with health claims, food for patients, foodmaterials, food materials with health claims, food materials for patients, food additives, food additives with health claims, food additives for patients, drinks, drinks with health claims, drinks for patients, drinking water, drinking water with health claims, drinking water for patients, medicinal agents, preparation raw materials, feeds, and feeds for domestic and/or wild animals under treatment.

For use as food, the lifespan extending agent of the embodiment of the present invention may be used as it is, or in the form of a diluted solution in water or the like, an oil suspension, or an emulsion. Further, the lifespan extending agent may be prepared by adding a carrier commonly used in food industry. The drinks may be non-alcohol drinks or alcohol drinks. Examples of non-alcohol drinks include carbonated drinks, non-carbonated drinks (such as fruit juice, and nectar) soft drink, sports drink, tea, coffee, and hot chocolate. The alcohol drinks may be in the form of, for example, beer, low-malt beer, third-category beer, *sake, umeshu,* wine, champagne, liqueur, *chuhai*, or medicated liquor.

For use as a food material or food additive, the lifespan extending agent of the embodiment of the present invention may be in the form of, for example, a tablet, a capsule formulation, a solid agent (such as a powder and a granule) dissolved in drinks, a semi-solid such as jelly, a liquid (such as drinking water), and a high-concentration solution diluted before use. Optional components, such as vitamins, carbohydrates, dyes, and flavoring agents commonly added to food may be appropriately mixed. The food may be given in any form, including a liquid and a solid. The lifespan extending agent may be given as a soft capsule formulation by being encapsulated in gelatin or the like. The capsule uses a gelatin coating prepared, for example, by dissolving the raw material gelatin in water, and by adding a plasticizer (such as glycerine, and D-sorbitol) to the gelatin solution.

Examples of dietary supplements and functional food include liquid food, semi-digested nutritional food, elemental dietary food, drinkable preparations, capsule formulations, and enteral nutritional supplements processed to contain, for example, sugars, fat, trace elements, vitamins, and flavoring agents. For improved nutritional balance and flavor, the food (for example, drinks such as sports drink, and nutritional drink) may be mixed with nutritional additives or compositions such as amino acids, vitamins, and minerals, or with spices, flavoring agents, or dyes.

The lifespan extending agent of the embodiment of the present invention is applicable to feeds for domestic animals, domestic chickens, and pets. For example, the lifespan extending agent may be mixed with dry dog food, dry cat food, wet dog food, wet cat food, semi-moist dog food, and feeds for poultry, or with feeds for domestic animals such as cows and pigs. The feed itself may be prepared by using an ordinary method.

The lifespan extending agent of the embodiment of the present invention can also be used for non-human animals, including, for example, domestic mammals such as cows, horses, pigs, and sheep; birds such as chicken, quail, and ostrich; reptiles, birds, and small mammals kept as pets; and hatchery fish.

The medicinal agent that exploits the lifespan extending effect of the lifespan extending agent of the embodiment of the present invention may be used alone, or may be orally, transnasally, percutaneously, or intravenously administered as a preparation in a suitable dosage form such as a liquid, a granule, a subtle granule, a powder, a tablet, a capsule formulation, a ball, an ointment, an adhesive skin patch, an atomizing agent, a spray, and an injection after being mixed with a suitable ordinary additive such as an excipient, a stabilizer, a preservative, a binder, and a disintegrant.

Organic or inorganic solids, semi-solid or liquid carriers, solubilizers, or diluents for medicinal use suited for oral administration, nasal administration, percutaneous administration, or intravenous administration may be used for preparing the composition of the embodiment of the present invention as a medicinal agent. Usable as a carrier for a medicinal agent containing the composition of the embodiment of the present invention are water, gelatin, lactose, starch, magnesium stearate, talc, animal·vegetable oil, benzyl alcohol, gum, polyalkylene glycol, petroleum resin, coconut oil, lanolin, and all other carriers that have medicinal use. Further, stabilizers, wetting agents, emulsifiers, and salts for changing the osmotic pressure or maintaining a suitable pH for a compounding agent also may be appropriately used as an auxiliary medicinal agent.

The lifespan extending agent of the embodiment of the present invention also can be used for cosmetics. Use of a soluble film for the preparation of cosmetics and other products has become common over the last years. For example, edible soluble films have been used as flavor films that hold ingredients such as a flavoring agent for refreshing purposes or for preventing a bad breath. Other applications based on previous ideas include a mask that uses a cosmetic film holding a moisturizer or the like, and an emulsion produced by dissolving the lifespan extending agent in water.

It is also possible to use a soluble film proposed as having excellent solubility and film characteristics and being preferred for use as a wrapping material for food and drugs, or as a carrier that holds the active ingredients of food and drugs (JP-A-2007-91696).

The lifespan extending agent of the embodiment of the present invention thus has a wide range of applications, including food and drinks, food additives, drugs, quasi drugs, oral compositions, cosmetics, and feeds.

The present invention is described below in more detail based on examples. It should be noted, however, that the present invention is in no way limited by the following examples.

### Example 1

### Experiment: Lifespan Extending Effect of D-psicose and D-allose in Nematodes

### (I) Test Method

### (1) Test Animal

The wild-type N2 strain of the nematode Caenorhabditis elegans was used.

### (2) Medium

Complete S liquid medium (Sulston, J.E. and Brenner, S. (1974) Genetics 77, 95-104) was used for the culturing of the nematode. D-psicose or D-allose was added to the medium in 0.25% (14 mM) and 0.5% (28 mM). The Escherichia coli (E. coli) OP50 strain was added in 30 mg (wet weight/mL) to feed the worms. To exclude the effects of the germ cells on lifespan, the cell division inhibitor 2'-deoxy-5-fluorouridine (40 µM) was added.

### (3) Measurements of Nematode Survival Rate and Average Lifespan

Young worms, 4 days of age, were used for the measurements of survival rate and average lifespan. The prepared liquid medium was dispensed onto ten plastic petri dishes having a diameter of 3.5 cm (2 mL each). Ten nematodes were transferred to each petri dish (a total of 100 individuals), and cultured at 20°C. The samples were inoculated into a new medium every day until day 3, and every 2 to 3 days thereafter. Observations were made at the time of the inoculation, and the number of survived individuals was recorded.

### (II) Test Results

### (1) Nematode Survival Rate over Time

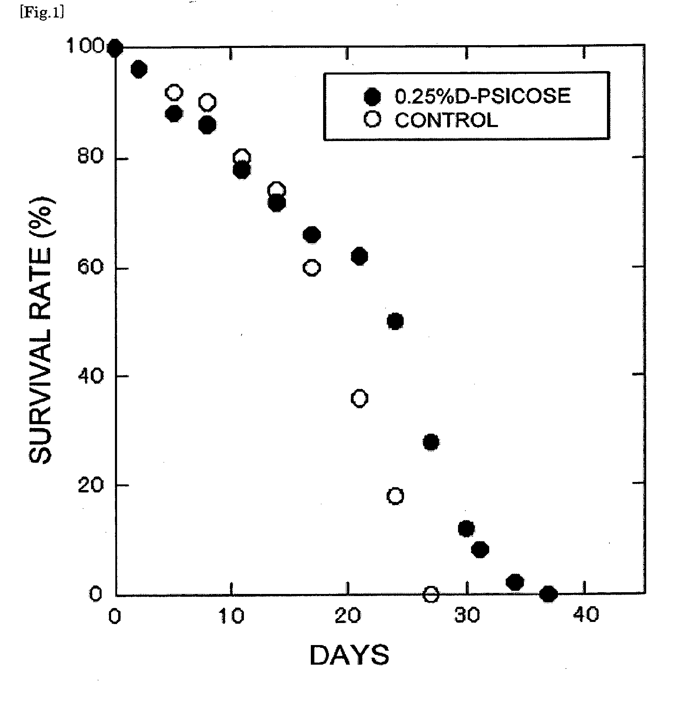

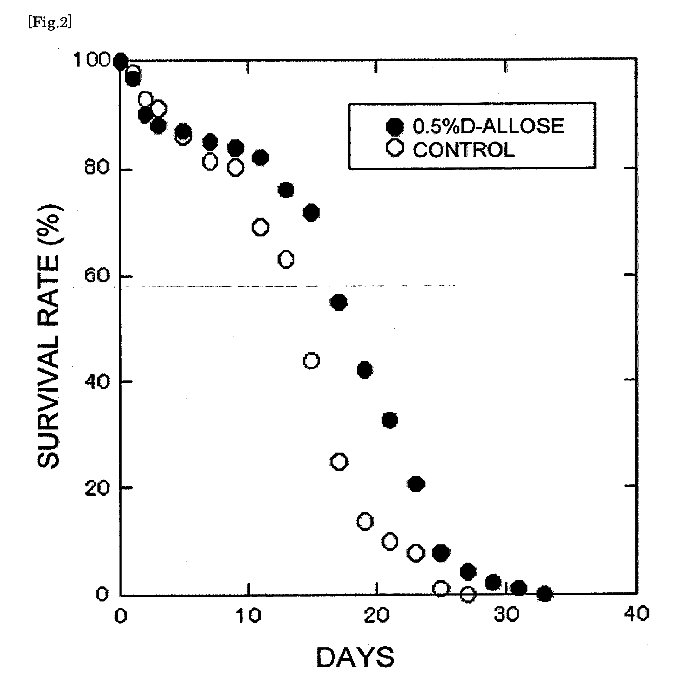

FIGS. 1 and 2 represent changes in nematode survival rate over time with addition of 0.25% and 0.5% D-allose. FIGS. 1 and 2 also show changes in the nematode survival rate of a control group.

### (2) Lifespan Extending Effect

The results of the comparison of the average lifespan calculated from days and nematodes survival rate are as follows.
1) The average lifespan after the treatment with 0.25% D-psicose was 18.9 days (15.8 days for the control), which represents a 20% increase.
2) The average lifespan after the treatment with 0.5% D-allose was 15.8 days (12.5 days for the control), which represents a 26% increase.

### (III) Results

The foregoing results confirmed a maximum of 26% increase in average lifespan after the treatment with the composition that contained the rare sugar as an active ingredient.

### Example 2

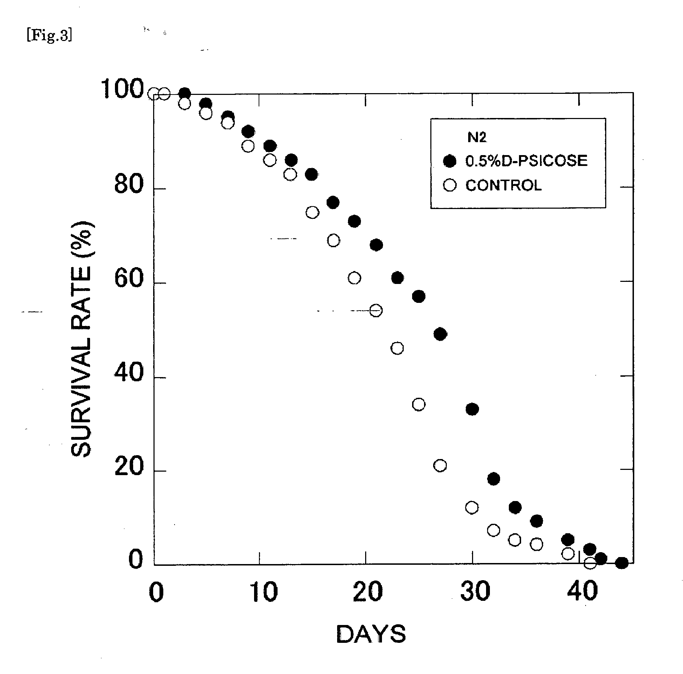

The wild-type N2 strain of the nematode Caenorhabditis elegans was cultured, and lifespan was measured in the same manner as in Example 1, except that the D-psicose was used in 0.5% (w/v). FIG. 3 shows a graph representing the survival rate of the nematode N2 after treatment with a D-psicose-containing composition. The average lifespan with 0.5% D-psicose was 27 days, longer than the 23-day lifespan of the D-psicose-free control.

### Example 3

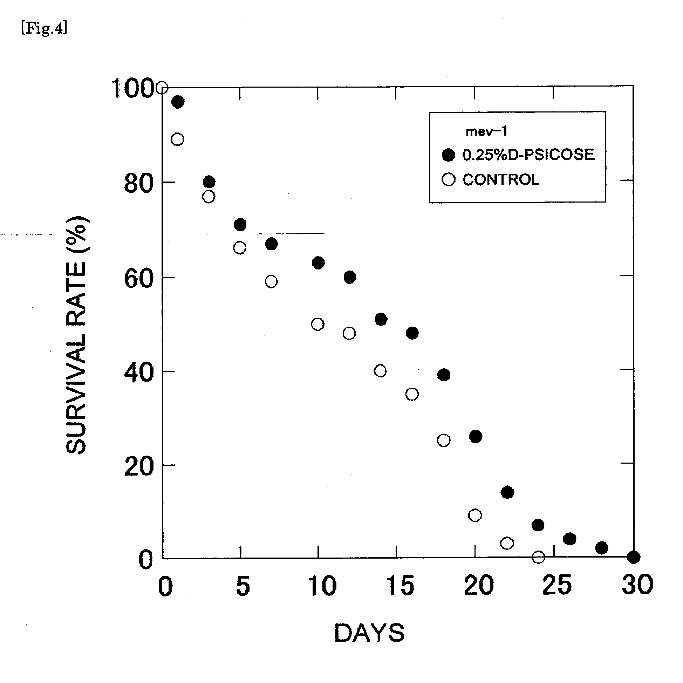

The oxygen sensitive mutant mev-1 of the nematode Caenorhabditis elegans was cultured, and lifespan was measured in the same manner as in Example 1, except that the D-psicose was used in 0.25%. FIG. 4 shows a graph representing the survival rate of the nematode mev-1 after treatment with a D-psicose-containing composition. The average lifespan with 0.25% D-psicose was 16 days, longer than the 12-day lifespan of the D-psicose-free control.

### Example 4

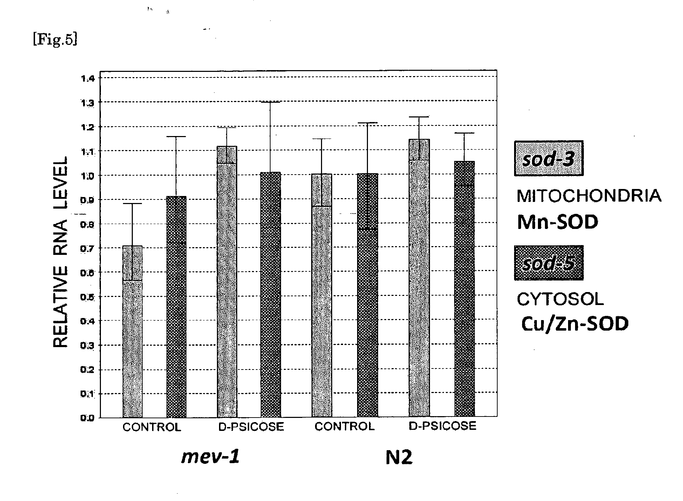

The wild-type N2 and the oxygen sensitive mutant mev-1 of the nematode Caenorhabditis elegans were cultured in liquid medium (10 mL) containing 0.5% (w/v) D-psicose. The medium was dispensed onto plastic petri dishes having a diameter of 10 cm, and approximately 1,000 nematodes were placed in each petri dish. The nematodes were cultured in the same manner as in Example 1 except for these differences, and the samples were collected on day 6 from the start of the culturing. FIG. 5 represents the results of the comparison of SOD mRNA gene expression levels. For comparison, the RNA gene levels of mitochondria Mn-SOD and cytosol Cu/Zn-SOD were measured. It can be seen that the both nematode strains have increased expression of the SOD gene.

### Example 5

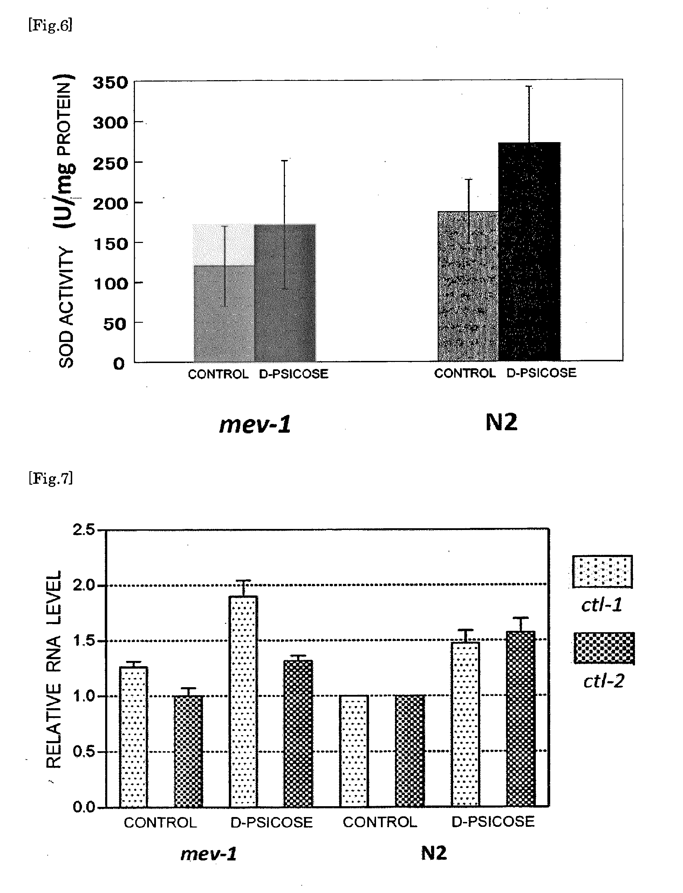

The wild-type N2 and the oxygen sensitive mutant mev-1 of the nematode Caenorhabditis elegans were grown in the same manner as in Example 4. The samples were collected on day 7 from the start of the culturing, and the SOD activity in the lysate was measured. The results are presented in FIG. 6. As is clear from the graph, the both nematode strains with D-psicose had increased SOD activity.

### Example 6

The wild-type N2 and the oxygen sensitive mutant mev-1 of the nematode Caenorhabditis elegans were cultured in the same manner as in Example 4. The samples were collected on day 6 from the start of the culturing, and the catalase mRNA gene expression levels were compared. The results are presented in FIG. 7. For comparison, the RNA gene levels of cytosol ctl-1 and peroxisome CTL-2 were measured. It can be seen that the both nematode strains have increased SOD gene expression.

### Example 7

The wild-type N2 of the nematode Caenorhabditis elegans was grown in the same manner as in Example 4. The samples were collected on day 7 from the start of the culturing, and the catalase activity in the lysate was measured. The results are presented in FIG. 8. As is clear from the graph, the both nematode strains with D-psicose had increased catalase activity.

### Example 8

D-psicose was given to rats, and the growing conditions were evaluated.

### Experiment Methods

Twenty-four male Wistar rats, 3 weeks of age, were divided into four groups (6 rats in each group), and grown for 10 months with free access to water and the following feeds.
(1) CE-2 (CLEA Japan) with 2.5% fructose (CE-2-F)
(2) CE-2 with 2.5% D-psicose (CE-2-P)
(3) Feed containing an isomerized sugar(HFCS) (sugar from isomerized sugar 28.5% + starch 28.5% (w/w): HFCS)
(4) Feed containing a rare sugar-containing syrup (RareSweet Co., Ltd.; sugar from rare sugar-containing syrup 28.5% + starch 28.5%: RSS)

After growth, the body weight of each rat was measured, and an autopsy was conducted for the weight measurement of the brain and other organs. The measured values were given as mean values ± standard deviation, and a t-test was applied.

### Results

The rat body weights (g) after growth were 452 ± 11 for the CE-2-F group, 436 ± 12 for the CE-2-P group, 417 ± 12 for the HFSC group, and 391 ± 17 for the RSS group. A significant body weight reduction was confirmed between the CE-2-F group and the CE-2-P group, and between the HFSC group and the RSS group. Comparisons of organ weights between the CE-2-F group and the CE-2-P group, and between the HFSC group and the RSS group did not find any significant difference in the brain and muscle.

As demonstrated above by the 10-month long-term growth experiment, the rare sugar-containing syrup containing rare sugars such as D-psicose and D-allose either alone or in combination suppresses obesity, without accompanied by reductions in brain weight or muscle mass often seen at old age. The result thus suggests the possibility that the syrup containing the rare sugars suppresses the age-related brain atrophy, and the reduction of muscle mass related to aging.

The present invention found novel characteristics of monosaccharides (rare sugars) found in trace amounts in nature, and developed a novel use of the rare sugars.

The present invention can provide a lifespan extending agent that contains rare sugars, preferably D-psicose and/or D-allose, usable as sweeteners, and that is safe, and can be easily ingested in a form that resembles food.

The present invention has high potential in industrial applications, because the invention provides a novel use of rare sugars produced by the recently developed method of producing rare sugars from monosaccharides abundant in nature.

A lifespan extending agent that is safe for animals, and can easily be ingested in a form that resembles foods. A lifespan extending method is also disclosed. In the lifespan extending agent, a rare sugar usable as a sweetener is contained as an active ingredient.

## Claims

1. An agent comprising a rare sugar usable as a sweetener as an active ingredient for use in extending lifespan by enhancing the production or activity of super-oxide dismutase (SOD) and catalase.

2. The agent for the use according to claim 1, wherein the rare sugar is D-psicose and/or D-allose.

3. The agent for the use according to claim 1, wherein the rare sugar is a sugar composition containing D-psicose and/or D-allose.

4. The agent for the use according to claim 3, wherein the rare sugar is a sugar composition containing 0.5 to 17.0% D-psicose and 0.2 to 10.0% D-allose with respect to the total sugar content.

5. The agent for the use according to claim 4, wherein the rare sugar is a mixed sugar produced by the conversion of raw material sugars D-glucose and/or D-fructose to include 0.5 to 17.0% D-psicose and 0.2 to 10.0% D-allose with respect to the total sugar content.

6. The agent for the use according to claim 4 or 5, wherein the rare sugar is a rare sugar-containing isomerized sugar usable as a sweetener.

## Patentansprüche

1. Mittel umfassend einen seltenen Zucker, der als ein Süßstoff verwendbar ist, als ein aktiver Inhaltsstoff für die Verwendung in der Verlängerung der Lebensspanne durch Erhöhung der Produktion oder der Aktivität von Superoxiddismutase (SOD) und Katalase.

2. Mittel für die Verwendung nach Anspruch 1, wobei der seltene Zucker D-Psicose und/oder D-Allose ist.

3. Mittel für die Verwendung nach Anspruch 1, wobei der seltene Zucker eine Zuckerzusammensetzung ist, die D-Psicose und/oder D-Allose enthält.

4. Mittel für die Verwendung nach Anspruch 3, wobei der seltene Zucker eine Zuckerzusammensetzung ist, die 0,5 bis 17,0% D-Psicose und 0,2 bis 10,0% D-Allose bezogen auf den Gesamtzuckergehalt enthält.

5. Mittel für die Verwendung nach Anspruch 4, wobei der seltene Zucker ein gemischter Zucker ist, der durch die Umwandlung der Rohmaterialzucker D-Glucose und/oder D-Fructose hergestellt ist, um 0,5 bis 17,0% D-Psicose und 0,2 bis 10,0% D-Allose bezogen auf den Gesamtzuckergehalt zu beinhalten.

6. Mittel für die Verwendung nach Anspruch 4 oder 5, wobei der seltene Zucker ein isomerisierter Zucker ist, der einen seltenen Zucker enthält, der als ein Süßstoff verwendbar ist.

## Revendications

1. Agent comprenant un sucre rare utilisable comme édulcorant ainsi que comme principe actif pour une utilisation dans l'extension de durée de vie par l'amélioration de la production ou de l'activité de la superoxyde dismutase (SOD) et de la catalase.

2. Agent pour l'utilisation selon la revendication 1, dans lequel le sucre rare est le D-psicose et/ou le D-allose.

3. Agent pour l'utilisation selon la revendication 1, dans lequel le sucre rare est une composition de sucre contenant le D-psicose et/ou le D-allose.

4. Agent pour l'utilisation selon la revendication 3, dans lequel le sucre rare est une composition de sucre contenant 0,5 à 17,0% de D-psicose et 0,2 à 10,0% de D-allose par rapport à la teneur totale en sucre.

5. Agent pour l'utilisation selon la revendication 4, dans lequel le sucre rare est un sucre mélangé produit par la conversion de sucres de matières premières D-glucose et/ou D-fructose de manière à inclure 0,5 à 17,0% de D-psicose et 0,2 à 10,0% de D-allose par rapport à la teneur totale en sucre.

6. Agent pour l'utilisation selon la revendication 4 ou 5, dans lequel le sucre rare est un sucre isomérisé contenant un sucre rare utilisable comme édulcorant.
